# EUROPEAN PATENT SPECIFICATION

(11) **EP 2 252 580 B1**
(45) Date of publication and mention of the grant of the patent: **20.05.2015**
(21) Application number: 09707391.0
(22) Date of filing: 02.02.2009
(51) Int. Cl.: C07C 303/06, C07C 303/32, C07C 309/17

(54) **PROCESS FOR THE PRODUCTION OF ALFA-SULFO FATTY ACID ESTERS AND THEIR SALTS**
VERFAHREN ZUR HERSTELLUNG VON ALPHA-SULFO-FETTSÄUREESTERN UND IHREN SALZEN
PROCÉDÉ DE PRODUCTION D'ESTERS D'ACIDES ALFA-SULFO GRAS ET DE LEURS SELS

(30) Priority: 05.02.2008 US 26174 P
(43) Date of publication of application: 24.11.2010
(73) Proprietor: Desmet Ballesta SPA, 20138 Milano (IT)
(72) Inventor: ADAMI, Icilio, I-20049 Concorezzo (IT); FORCELLA, Alessandro, I-23887 Olgiate Molgora (LC) (IT); GIUSTI, Luigi, I-20138 Milano (IT); ROBERTS, David W., Merseyside CH63 9LD (GB)
(74) Representative: Nederlandsch Octrooibureau
(86) International application number: PCT/EP2009/051125
(87) International publication number: WO 2009/098176

(56) References cited:
- US-A- 4 021 460
- US-B1- 6 657 071

## Description

### FIELD OF THE INVENTION

The invention relates to a process for producing α-sulfo fatty acid esters and their salts from fatty acid esters with an improved yield and less by-products.

### BACKGROUND OF THE INVENTION

Fatty acid ester sulfonates are commercially important products and are used as wetting agents and detergent components because of their excellent surface-active properties, resistance to hard water and their biodegradability. They are manufactured by sulfonating fatty acid esters such as but not limited to fatty acid methyl esters (FAME) with a molar excess of diluted gaseous sulfur trioxide and allowing the adduct to age. After the ageing stage, methanol may be added to re-esterify free carboxylic acid groups while using the acid still present as esterification catalyst. This acid is then neutralized by the addition of caustic. Because the sulfonate salt thus obtained tends to be too dark in colour for commercial use in detergents, it can be bleached with for instance aqueous hydrogen peroxide. After having been dried, flaked and ground, the resulting powder is ready for inclusion into detergent compositions.

As disclosed in US Patent No. 3,169,142, continuous sulfonation processes using gaseous sulfur trioxide preferably employ a reactor comprising a bundle of tubes on the inside of which the fatty acid alkyl ester flows downwards under conditions of high turbulence, while the shell side of the reactor acts as a heat exchanger controlling the temperature of the reaction mixture. Details of such a multi-tubular reactor are disclosed in British Patent Specification No. 2 043 067.

In the literature, there is a general consensus that the sulfonation of FAME involves a series of consecutive reactions. In the first of these reactions, sulfur trioxide attaches itself to the carbonyl oxygen atom of the FAME to form an intermediate that rearranges to the methyl ester of a mixed anhydride according to:

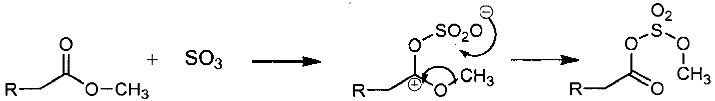

The formation of this mixed anhydride methyl ester activates the carbon atom alpha to the carbonyl which enables free SO₃ to form another intermediate that is both a mixed anhydride methyl ester and a sulfonate:

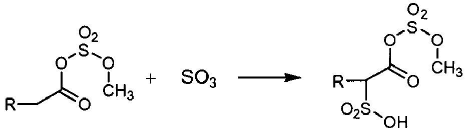

During ageing, this latter intermediate will loose a molecule of SO₃ but since both reactions illustrated above are much faster than the reactions involved in the ageing process, a reaction starting with a molar ratio of SO₃ to FAME of less than 2.0 will leave some free FAME.

One of the reactions involved in the ageing process is a slow release of SO₃ leading to the formation of the α-sulfo methyl ester according to: 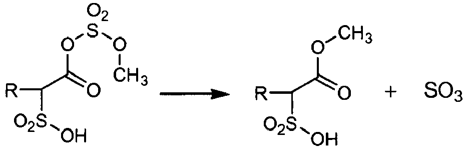 This α-sulfo methyl ester, also called methyl ester sulfonate (MES), is the product aimed for.

Another reaction of the sulfonated mixed anhydride methyl ester is a disproportionation reaction leading to a dimethylated mixed anhydride sulfonate and a non-methylated mixed anhydride sulfonate according to:

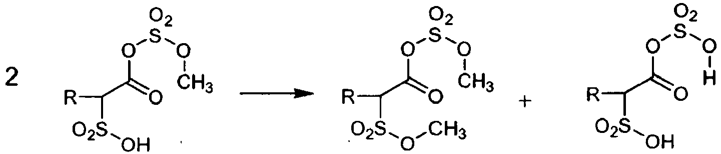

The non-methylated mixed anhydride sulfonate may loose a sulfur trioxide molecule to form a sulfonated fatty acid, which on re-esterification with methanol yields MES according to:

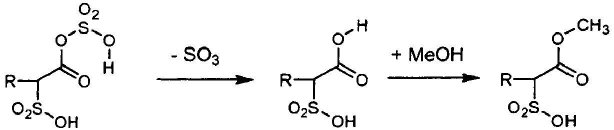

Because neither of the above reactions will be complete, both the residual non-methylated mixed anhydride sulfonate and the residual fatty acid sulfonate will yield the di-salt of an α-sulfo fatty acid on neutralization according to: 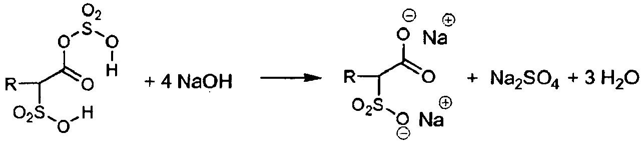 and

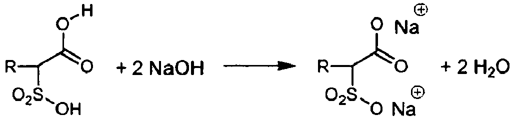

The di-salt can also be formed from iso-MES, an α-sulfo fatty acid on which the sulfo group is esterified with methanol. However, this formation is preferably prevented by prior re-esterification causing the mixed anhydride sulfonate to form MES and methyl sulfate rather than iso-MES and di-salt after neutralization as indicated below:

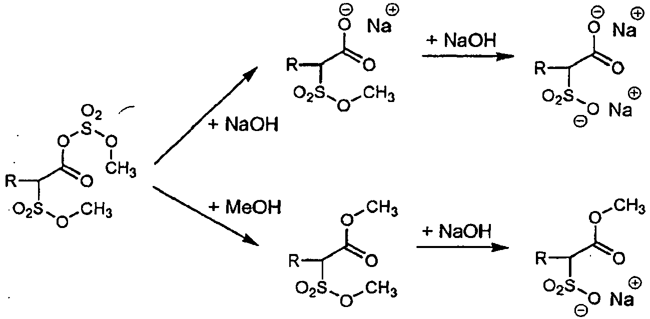

The above summary of what generally happens during sulfonation, ageing, re-esterification and neutralization is a simplification. The above summary does not mention either what reactions are involved in the formation of strongly colored compounds because this aspect of MES production is hardly understood.

Accordingly, no explanation can be given for the observation disclosed in US Patent No. 4,021,460 that the addition of less than 10 parts by weight of an inorganic sulfate to 100 parts (claim 1 as granted discloses 0.5 to 10 parts by weight of an inorganic sulphate to 3 parts) of saturated fatty acid alkyl ester before this is sulfonated by 1.0 to 2.0 moles of SO₃ per mol of said fatty acid ester causes the amount of by-product to decrease with the resultant lowering of the coloration of the MES thus produced. The inorganic sulfate must be selected from the group consisting of sodium, calcium, ferric, aluminium and ammonium sulfates and they must be mixed into the MES as an anhydrous powder.

Similarly, US Patent No 6,657,071 discloses the use of color inhibitors in a method for the production of α-sulfo fatty acid ester salts comprising a sulfonation step for bringing a fatty acid alkyl ester into contact with a sulfonating gas in the presence of a color inhibitor to sulfonated the fatty acid alkyl ester.

According to said patent: "As coloring inhibitor, inorganic sulfuric acid salts or organic acid salts containing monovalent metal ions and having a mean particle size of 250 µm or less are preferable. The inorganic sulfuric acid salts are not particularly limited as far as they are powdery anhydrous salts containing monovalent ions. For example, sodium sulfate, potassium sulfate, lithium sulfate etc. are exemplified. The mean particle diameter of the inorganic salts is 250 µm or less, and preferably 100 µm or less. By setting it to 250 µm or less, the contact area with the raw material becomes greater to increase the dispersibility. The inorganic sulfuric acid salts have high coloring inhibition effects and are mostly inexpensive and further are components which can be blended with detergents so that they do not have to be finally removed from the α-sulfo fatty acid alkyl ester salts (commercial products).

As the organic acid salts, sodium formate, potassium formate, sodium acetate, etc. are preferable. The mean particle diameter of the organic acid salt is also set to 250 µm or less, or preferably 100 µm or less from the viewpoint at an increased contact area with the raw material."

The use of color inhibitors is also disclosed in Japanese Patent Application No. 2000128852 (published May 9, 2000), Japanese Patent Application No. 2000128855 (published May 9, 2000) and Japanese Patent Application No. 9278740 (published October 28, 1997).

However, both the inorganic sulfates disclosed in US Patent No. 4,021,460 and the color inhibitors disclosed in US Patent No 6.657,071 have the disadvantage that they do not dissolve in the FAME raw material. Instead, they must be finely powdered, mixed with the FAME and then kept in suspension. Therefore they can only be used in agitated vessels and not in multi-tubular flow reactors. Consequently, existing plants will face extensive and therefore expensive modifications if they were to be adapted to the use of the inorganic sulfates and/or color inhibitors as mentioned above.

US Patent No. 4,404,143 discloses the preparation of highly concentrated aqueous solutions salts of sulfonated fatty acid esters, wherein the sulfonated fatty acid ester is neutralised with aqueous NaOH solution in the presence of a C₁-C₄ alcohol to form an acidic neutralised product, the latter being converted into a alkaline neutralised product by the addition of more NaOH. It is preferred that prior to the neutralization step, the sulfonated fatty acid ester is bleached, wherein any excess hydrogen peroxide is decomposed during neutralization.

US Patent No. 4,695,409 discloses a process wherein the formation of disalts is prevented. The process involves sulfonation followed by transesterification with a lower alcohol. Subsequently, the product is worked up in the usual manner, i.e. neutralization followed by bleaching or bleaching followed by neutralization. The use of inorganic sulfates is not disclosed.

US Patent No. 5,668,982 discloses the manufacture of salts of sulfonated fatty acid esters, wherein the fatty acid alkyl ester is sulfonated. Subsequently, the sulfonated fatty acid alkyl ester is reacted with an alcohol and then neutralised with a metal alkoxide in an anhydrous medium, preferably methanol. The use of e.g. inorganic sulfates is not disclosed.

US Patent No. 5,475,134 discloses a further method for the preparation of the salts of sulfonated fatty acid esters, said method being said as having the advantage that undesired formation of by-products is minimised by over-neutralization of the crude sulfonation product to a pH of at least 10 with an alkoxide solution containing not more than 1 wt.% of water followed by re-neutralization in an anhydrous medium to a pH of 5 to 9.

### OBJECTS OF THE INVENTION

It is an object of the invention to overcome the disadvantages of the processes according to the prior art.

It is also an object of the invention to improve the yield of sulfonated fatty acid methyl esters with respect to the fatty acid methyl ester starting material.

It is a further object to produce lightly colored sulfonated fatty acid methyl esters.

It is another object of the invention to avoid the need to grind the inorganic sulfates before they are dissolved in the organic reaction medium.

It is also an object of the present invention to minimise any modification that might be required to existing plants when adopting the process of the invention.

These and other objects of the invention will become more apparent in the discussion below.

### SUMMARY OF THE INVENTION

It has surprisingly been found that inorganic sulfates including acid sulfates are highly effective in increasing the MES yield during the sulfonation of FAME when said sulfates are dissolved in the reaction mixture resulting from the sulfonation of fatty acid alkyl esters, *i.e.* after the sulfur trioxide has been added and before said reaction mixture is allowed to age, and moreover that introducing an additional ageing step after the neutralization of the reaction mixture increases the MES yield even further.

Accordingly, the present invention discloses a process for the production of fatty acid alkyl ester sulfonates comprising the steps of:
a) sulfonating a fatty acid methyl ester with gaseous sulfur trioxide in a continuous process thereby generating a crude sulfonation product comprising more than 15 % by weight of residual fatty acid methyl esters;
b) dissolving an amount of less than 10% by weight of inorganic sulfates, calculated on the weight of the crude sulfonation product, into the crude sulfonation product resulting from step a);
c) ageing the solution resulting from step b);
d) re-esterifying the aged product resulting from step c) by the addition of methanol;
e) neutralising the re-esterified product resulting from step d); and
f) bleaching the neutralised product resulting from step e), wherein up to 25% by weight of the solution resulting from step a) and/or step b) is recycled to and mixed into the fatty acid methyl ester stream being sulfonated in step a).

### BRIEF DESCRIPTION OF THE DRAWINGS

Figure 1 represents a flow diagram illustrative of various embodiments of the process according to the present invention.
Figure 2 shows a flow diagram of a preferred embodiment of the process according to the present invention.

### DETAILED DESCRIPTION OF THE INVENTION

The verb "to comprise" as is used in this description and in the claims and its conjugations are used in its non-limiting sense to mean that items following the word are included, but items not specifically mentioned are not excluded. In addition, reference to an element by the indefinite article "a" or "an" does not exclude the possibility that more than one of the element are present, unless the context clearly requires that there is one and only one of the elements. The indefinite article "a" or "an" thus usually means "at least one".

The present invention relates to the production of α-sulfo fatty acid methyl esters (methyl ester sulfonates or MES) by using fatty acid methyl esters (FAME) as starting material. The FAME may originate from animal or vegetable oils and fats and their fatty acid chains will contain from 10 to 20 carbon atoms. They must be hydrogenated to an iodine value of less than 0.5 (g I₂ per 100 g FAME), and preferably less than 0.2 (g I₂ per 100 g FAME).

The process according to the invention can be executed discontinuously in a batch process but it is preferably executed continuously. In the latter instance, use can preferably be made of a tubular, falling film reactor allowing superior temperature control of the reaction mixture when for instance a multitude of tubes is fitted inside a shell being supplied with a heat exchange medium such as water.

The temperature of the reaction mixture can thus be controlled within a range of 50°C to 80°C, preferably from 55°C to 60°C. To facilitate this control, the FAME being distributed over the tubes is preheated to a temperature of 50°C to 65°C, preferably to a temperature of 52°C to 50°C; similarly the sulfur trioxide is preheated to a temperature of 45°C to 60°C, preferably to a temperature of 48°C to 55°C.

In such a reactor, the FAME is allowed to flow as a film down the tube wall while sulfur trioxide (SO₃) is flowing co-currently along said film. This SO₃ is preferably diluted with dry air (dew point -60°C) to 4 to 8 % by volume and preferably to 5 to 6 % by volume. The amount of SO₃ to be used in the sulfonation of FAME corresponds to a molar excess with respect to the FAME of 10 to 25% and preferably 15 to 18%.

During the sulfonation reaction, the viscosity of the reaction mixture increases substantially. Consequently, the film at the upper end inside the tubular reactor will be much thinner than at the lower end of said tube. A more even distribution of the reaction mixture over the wall of said tube is attained in the process according to the invention in which an amount of up to 25% by weight of the reaction mixture leaving the tube at its lower end is recycled to its upper end by mixing said amount with the FAME starting material. Dissolving the viscous sulfonation reaction product into FAME causes the viscosity of the latter to increase and flow less rapidly down the upper part of the tubular, falling film reactor. It thereby increases the effectiveness of said reactor.

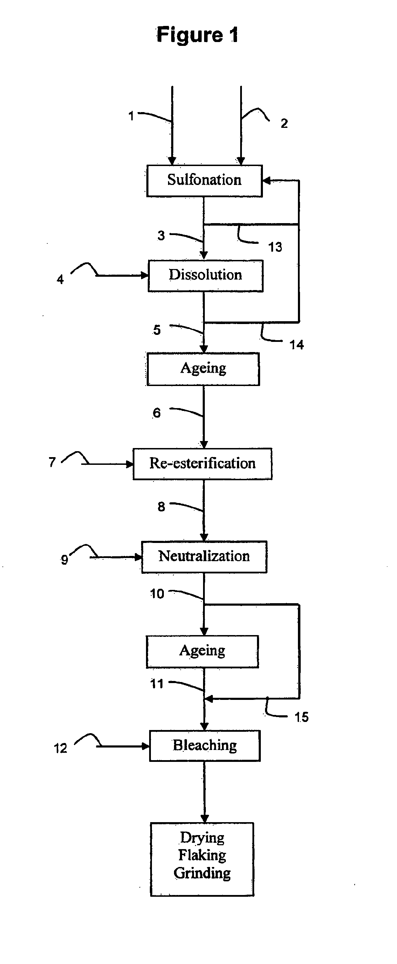

In another embodiment of the process according to the invention, the sulfonation reaction product is not recycled as such but only after an amount of less than 10 % by weight of inorganic sulfate has been dissolved in said reaction product. Like the previous embodiment comprising the recycling of the sulfonation product as such, this embodiment which comprises the recycling of the solution of inorganic sulfate in said sulfonation product has been shown schematically in Figure 1.

During this sulfonation reaction, the SO₃ will react with the FAME under formation of the sulfonated mixed anhydride methyl ester. Given the relatively small molar excess of SO₃ and the short residence time in the tubular reactor which prevents extensive ageing, the reaction mixture leaving the tubular reactor will contain substantial amounts of residual FAME. Said amounts may be as high as 15 % by weight or more, calculated on the total weight of the reaction mixture. If the sulfonation were to be carried out in a batch reactor, the residence time would be much longer than in a tubular, falling film reactor and consequently, sulfonation and ageing would proceed simultaneously. The SO₃ liberated during ageing would then be available for further sulfonation causing the residual FAME content at the end of the sulfonation step a) after all SO₃ has been added to the reactor, to be well below 15 % by weight, calculated on the total weight of the reaction mixture.

In step b) of the process according to the invention, inorganic sulfates such as sodium sulfate or magnesium sulfate are now dissolved in the sulfonation reaction mixture. The sulfates are preferably anhydrous but good results have also been obtained with sulfates containing water of crystallization. They preferably have a mean particle size of less than 100 µ but this is far from mandatory since they do not have to be kept in suspension; they just dissolve more rapidly when they have a small size. Accordingly, it is often not necessary to grind the inorganic sulfates before dissolving them in the sulfonation reaction mixture. The amount of sulfates to be dissolved in the sulfonation mixture is less than 10 % by weight (but more than 0.1 % by weight) of said mixture and preferably 2 to 5 % by weight.

The chemical nature of the sulfates is not that critical but the use of sulfates that comprise or that are acidic sulfates such as sodium hydrogen sulfate has been found to be advantageous in ensuring an increased conversion of FAME into MES. The use of magnesium sulfate, acidic magnesium sulfate or a mixture thereof has also been found to be particularly advantageous. The sulfates dissolved in the sulfonation reaction mixture partake in the subsequent reactions. They may well liberate their sulfur oxide, which can then act as sulfonating agent and their metal ion may act as neutralising agent and thus save on the alkali to be used in step e) of the process according to the invention. The sulfates may be anhydrous or may comprise water of crystallization.

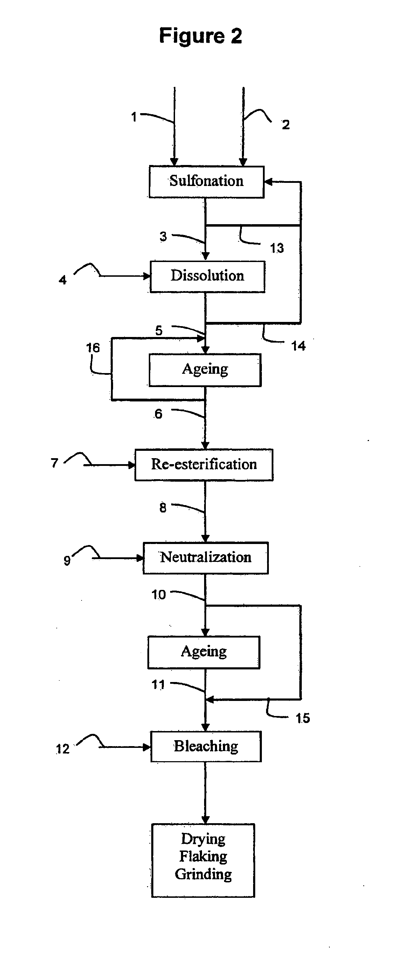

According to a preferred embodiment of the present invention, (part of) the inorganic sulfates are mixed in a side stream formed from 1 to 30 wt.% of the total aged product resulting from step c), preferably 5 to 25 wt.%, wherein the formed mixture is fed to ageing step c). The advantage is that acidic material entering the ageing step c) immediately encounters the color depressing effect of already dissolved sulfate. Hence, it is preferred that the process according to the present invention comprises a step b1) wherein at least a part, preferably at least 5 to 30 wt.%, of the inorganic sulfates are mixed with a side stream formed from 1 to 30 wt.% of the total aged product resulting from step c), preferably 5 to 25 wt.%, wherein the formed mixture is recycled to ageing step c). This feature is shown in Figure 2.

In step c) of the process according to the invention, the solution of inorganic sulfates in the sulfonation reaction mixture is aged for a period of time, preferably for 2 hours but prior to this ageing, the temperature of said solution is raised to 80°C. In a continuous process, this rise in temperature can be easily achieved by pumping the reaction mixture through a heat exchanger to either a plug-flow reactor ensuring a residence time of some 2 hours or to one of two reaction vessels that are used intermittently.

During the ageing process, the sulfur trioxide that is liberated by the decomposition of the sulfonated mixed anhydride methyl ester will react with residual FAME but eventually, the excess of sulfur trioxide will be liberated as free gas. This gas is removed by maintaining a slight vacuum in the ageing vessel and collecting the vent gases for recycling. Neither the length of the ageing period nor the temperature during ageing are highly critical. If a somewhat lower temperature is used, the ageing duration should be somewhat extended. However, after the ageing, the temperature should preferably be lowered to 60°C for the re-esterification step d) of the process according to the invention. However, lowering the temperature is not mandatory; it only permits the re-esterification with methanol to be carried out at atmospheric pressure.

In said re-esterification step, a maximum amount of methanol of 3 % by weight of the reaction mixture is dispersed in the aged mixture resulting from step c). The resulting dispersion is allowed to react for a period of some 1 hour. Said mixture is sufficiently acidic to catalyse the esterification of free carboxyl groups with methanol so no esterification catalyst needs to be added to the dispersion. A plug flow reactor is the reactor type of choice in a continuous process. After said reaction period, the reaction mixture is exposed to vacuum to evaporate any free methanol left.

In step e) of the process according to the invention, the re-esterified product resulting from step d) is neutralised with alkali such as caustic soda. If caustic soda is used, it is preferably diluted with water to a strength of 30 % by weight before being allowed to react with said re-esterified product. Because the.neutralization is exothermic, the temperature of the reaction mixture is preferably controlled by using a loop comprising a mixer being fed with said re-esterification product and caustic soda and a heat exchanger that maintains the temperature of the reaction mixture below 70 °C. The amount of caustic to be used in the neutralization step e) of the process according to the invention is such that after neutralization a pH of 6.0 to 7.5, preferably 6.5 to 7.0 is reached.

According to a preferred embodiment according to the present invention, the neutralised product resulting from step e) is subjected to an additional ageing step before it is subjected to the bleaching step f) as illustrated in Figure 1. This further ageing process offers the advantage of further increasing the MES content of said product and diminishing bleaching requirement. For this further ageing process, a plug-flow reactor providing a residence time of about 1 to about 4 hours, preferably about 2 to about 3 hours, is the preferred equipment. There is no need to adjust the temperature of said product.

In bleaching step f) of the process according to the present invention, the neutralised product that may have undergone a further ageing treatment is bleached and among the various bleaching agents that have been described in the literature, the preferred bleaching agents are hydrogen peroxide and sodium hypochlorite. Said oxidising agent is normally supplied at 30 % by weight strength and there is not need to dilute said agent for the bleaching step f). During the bleaching process the temperature of the reaction mixture is maintained between 60°C and 80°C, preferably between 65°C and 70°C. If necessary, the pH of the reaction mixture is adjusted to a value between 6.5 and 7.0.

Finally, the bleached product is dried, flaked and ground according to methods that are known *per se* to those skilled in the art.

Figure 1 shows a preferred embodiment of the process according to the present invention wherein FAME **1** and gaseous sulphur trioxide **2** are fed to the sulfonation reactor. The crude sulfonation product **3** is then mixed with inorganic sulphate **4**, where after solution **5** is subjected to an ageing step. After the ageing step, the aged product **6** is contacted with a feed 7 comprising methanol. The esterified product **8** is then neutralised by adding a feed **9** comprising a base. After neutralisation, the neutralised product **15** is subjected to a bleaching step by contacting said neutralised product **15** with a feed **12** comprising a bleaching agent. As is indicated in Figure 1, the crude sulphonated product **3** and/or the solution of inorganic sulphate and crude sulfonated product **5** may be partly recycled (**13** and **14**, respectively) to the sulfonation step. As is disclosed above, it is preferred that the neutralised product **10** is subjected to a second ageing step and that the aged product is **11** is fed to the bleaching step where it is contacted with a feed **12** comprising a bleaching agent.

Figure 2 shows another preferred embodiment of the present invention. Figure 2 is identical to Figure 1, provide that it includes the preferred embodiment of the (partly) recycle of the aged product **6** together with inorganic sulphate (cf. **16**).

### EXAMPLES

### MATERIALS AND METHODS

The *fatty acid methyl ester* (FAME) used during the experiments to be described in the examples was a palmitic acid methyl ester with properties as shown in Table 1:

**TABLE 1**

| **Property** | **Unit** | **Value** |
|---|---|---|
| Acid value | mg KOH /g | 0.23 |
| Unsaponifiable | % by weight | 0.52 |
| Moisture | ppm | 360 |
| Iodine value | g I₂ per 100 g | 0.09 |
| Average relative molecular mass | | 269.5 |
| Fatty acid composition | weight % | |
| C14 and below | | 0.35 |
| C16 | | 99.55 |
| C18 and above | | 0.1 |

The *methanol* used in the examples had a water content below 1 000 ppm as determined by a Karl Fisher titration.

For the *continuous sulfonation reactions,* a falling film reactor was used that was 6 m tall and that had an internal diameter of 2.5 cm. The reactor was surrounded by a water jacket that was divided in three sections each of which formed part of a circulation loop with independent temperature control at 90°C. FAME was fed to the top of the reactor at a temperature of 55°C together with gaseous sulfur trioxide that was diluted in dry air to 5 % by volume; the temperature of the gas mixture was controlled at 52°C. Unless otherwise stated, the molar ratio of the sulfur trioxide to the FAME was 1.15 : 1.

The *acid value* of the reaction product and intermediate products is determined by titration. An amount of approx. 0.4 g is weighed to the nearest mg, dissolved in 50 mL of neutralised isopropanol and titrated with 0.1 N NaOH using phenolphthalein as indicator.

The *residual FAME content* is determined by extracting an alkaline solution of the sample in aqueous ethanol (50/50) with n-hexane or petroleum ether (40-60°C). To this end, a sample of 1 to 1.5 g is weighed to the nearest mg and dissolved in a solution of 0.2 g sodium bicarbonate in 100 mL aqueous ethanol (50/50) and extracted three times with 100 mL of n-hexane or petroleum ether. The extracts are combined, washed with 50 mL aqueous ethanol to which a few drops of caustic soda have been added and then with successive amounts of 30 mL aqueous ethanol until neutral to phenolphthalein. Then the washed extract is dried over anhydrous sodium sulphate and evaporated to dryness after which its weight is determined.

The *color of the product as expressed in °Klett* is determined by using a Klett-meter and a 5 % solution and a 4 cm cuvette. For MES, the solvent is isopropanol but water is used as solvent when determining paste colour.

The *Total Active Matter* is determined by potentiometric titration. If this is carried out in a slightly acidic medium (pH = 3), only the concentration of sulphonic acid groups will be determined but in slightly alkaline medium (pH = 10), the carboxylic acid groups are determined as well. The Total Active Matter is then calculated by taking the relative molecular masses into account.

### EXAMPLE 1

FAME and SO₃ are fed to the falling film reactor is at a rate of 30 kg/h and 10.3 kg/h respectively; this corresponds to a molar excess of 16%. The reaction product is collected and pumped through a heat exchanger that raises its temperature to 80°C, to a first agitated vessel that provides the reaction mixture with an average residence time of 25 min. Anhydrous sodium sulfate with a particle size below 100 µm is also added continuously to this first vessel and dissolved in the sulfonation reaction mixture. From this first vessel the partially aged reaction mixture comprising sodium sulfate is passed to a second agitated vessel providing an average residence time of 25 minutes and from there to a third agitated vessel providing an average residence time of 55 minutes.

Then the product is cooled to 60°C by passing it through a jacketed tube to an agitated re-esterification vessel into which methanol is also added at a rate of 1.0 kg per hour and that provides the reaction mixture with an average residence time of 20 minutes. After the re-esterified mixture has been passed through a flash chamber maintained at a pressure of 0.1 atm. (about 101 hPa) at 85°C, the product is continuously neutralised by the addition of 17 kg per hour of a 29 % (w/w) of sodium hydroxide. To ensure good mixing, the reaction mixture is recirculated in a loop at a rate of 300 kg/h. The resulting paste is subjected to a subsequent ageing process which is also a continuous process with a flow rate of some 58 kg/h. It is carried out in a non-agitated vessel encouraging a plug flow with a residence time of 1.5 hours.

An amount of 60 kg of the neutralised product is bleached in an agitated, jacketed batch reactor with a volume of 160 L. Before the hydrogen peroxide is added to the reactor, the paste is heated to 80°C by passing hot water through the vessel jacket. An amount of 6.3 kg of hydrogen peroxide in water (30 % w/w) is added slowly over a period of 4 hours. The total bleaching time is 8 hours.

Samples were taken at several stages and their analytical data have been listed in Table 2.

**Table 2**

| **Process step after which sample is taken** | **Flow rate** (kg/h) | **Temp.** (°C) | **Residual FAME** (% by weight) | **Color** (°Klett) |
|---|---|---|---|---|
| Sulfonation | 40.3 | 60 | --- | 220 |
| First ageing | 41.1 | 80 | 14.80 | 1750 |
| Second ageing | 41.1 | 80 | 7.35 | 2300 |
| Third ageing | 41.1 | 80 | 3.25 | 2800 |
| Re-esterification | 42.1 | 60 | 2.79 | 2400 |
| Neutralization | 58.0 | 55 | 2.50 | 1680 |
| Post-ageing | 58.0 | 55 | 2.50 | 1400 |
| Batch bleaching | --- | 80 | 2.25 | 55 |

Table 2 clearly shows that the residual FAME content of the reaction mixture decreases during the successive ageing steps from 14.80 % by weight after the first ageing step during which the sodium sulfate is dissolved in the reaction mixture to 3.25 % by weight after the third ageing step. It also illustrates that the post-ageing step is quite effective in lowering the color before any bleaching agent is added.

### EXAMPLE 2

In this example, the effects of recycling sulfonated product and of dissolving various amounts of sodium sulfate in the sulfonated product being recycled are investigated. Accordingly, a fatty acid methyl ester with properties as listed in Table 1 is sulfonated as described above; a 300 g sample of the sulfonated product is taken for analysis (Sample A).

A stream of 3 kg/h of the sulfonated product is diverted and mixed with the FAME raw material and the mixture is then fed to the falling film reactor with a rate of 33 kg/h. After a steady state had been established, another 300 g sample of the sulfonation product leaving the reactor was taken (Sample B).

In this experiment, sodium sulfate is dissolved in the sulfonation product corresponding to Sample A. Accordingly, the sulfonation product leaving the reactor is mixed with 2 kg/h of anhydrous sulfate and 3 kg/h of the ensuing solution is mixed with the FAME raw material before this mixture is fed to the sulfonation reactor at a rate of 33 kg/h. A 300 g sample is taken at the outlet of said reactor (Sample C), thus before fresh sodium sulfate is dissolved into the product.

In the final experiment of this example, the sodium sulfate content of the material being sulfonated is doubled. Accordingly, an amount of 6 kg/h is mixed with the FAME raw material instead of the 3 kg/h mentioned in the previous experiment and the flow rate through the falling film reactor is increased to 36 kg/h. The sample taken after sulfonation and before sodium sulfate dissolution is denoted as Sample D. Pertinent data are listed in Table 3

**TABLE 3**

| **Sample** | **Amount of product being recycled** | **Sodium sulfate content** | **Residual FAME** |
|---|---|---|---|
| | (% by weight on FAME) | (% by weight) | (% by weight) |
| A | 0 | 0 | 3.45 |
| B | 10 | 0 | 2.80 |
| C | 10 | 0.5 | 1.75 |
| D | 20 | 1.0 | 1.00 |

Table 3 shows that recycling 10 % by weight of sulfonation product to the sulfonation reactor inlet causes the residual FAME content to decrease from 3.45 to 2.80 % by weight. Including 0.5 % by weight (calculated on the FAME being sulfonated) in the sulfonation reaction mixture leads to a further decrease of the residual FAME content from 2.80 to 1.75 % by weight and doubling the amount of sodium sulfate decreases said residual content to only 1.00 % by weight.

### EXAMPLE 3

This example shows the effect of ageing time and sodium sulfate concentration in the residual FAME in a batch ageing process. In separate batches, 300 g of the sulfonation product corresponding to Sample A in Example 2 were aged for 60 or 120 minutes without sodium sulfate or with 2 % by weight of sodium sulfate. The results are shown in Table 4.

**TABLE 4**

| **Ageing time** | **Sodium sulfate content** | **Residual FAME** |
|---|---|---|
| (minutes) | (% by weight) | (% by weight) |
| 60 | 0 | 4.05 |
| 120 | 0 | 3.05 |
| 60 | 2 | 2.85 |
| 120 | 2 | 1.00 |

In the absence of sodium sulfate, increasing the ageing time of the sulfonation reaction mixture from 60 to 120 minutes decreases the residual FAME from 4.05 to 3.05 % by weight. Dissolving 2 % by weight of anhydrous sodium sulfate in the sulfonation reaction mixture while maintaining the ageing time at 60 minutes decreases the residual FAME content from 4.05 to 2.85 % by weight and a further decrease to only 1.00 % by weight results when 2 % by weight of anhydrous sodium sulfate is dissolved in the sulfonation product and the ageing time is extended to 120 minutes.

### EXAMPLE 4

In this example, magnesium sulfate containing water of crystallization (MgSO₄.7 H₂O) and anhydrous sodium hydrogen sulfate (NaHSO₄) are used to improve the conversion of FAME to MES. The experiments are executed in the same way as Example 1 except that instead of the anhydrous sodium sulfate, 800 g/h of magnesium sulfate or sodium hydrogen sulfate is dissolved in the sulfonation reaction mixture after this has been heated to a temperature of 80°C. The particle size of the magnesium sulfate was less than 100 µ and it dissolves so rapidly in said reaction mixture that no particles could be discerned in the solution leaving the first ageing vessel. The sodium hydrogen sulfate also dissolves very fast. Pertinent data are summarised in Table 5.

**TABLE 5**

| **Process step after which sample is taken** | **Flow rate** (kg/h) | **Temps** (°C) | **Residual FAME** (% by weight) | | **Color** (°Klett) | |
|---|---|---|---|---|---|---|
| | | | MgSO₄ | NaHSO₄ | MgSO₄ | NaHSO₄ |
| Sulfonation | 40.3 | 60 | --- | --- | 205 | 195 |
| First ageing | 41.1 | 80 | 14.10 | 15.50 | 1850 | 1680 |
| Second ageing | 41.1 | 80 | 6.95 | 8.00 | 2450 | 2100 |
| Third ageing | 41.1 | 80 | 3.58 | 3.78 | 2950 | 2600 |
| Re-esterification | 42.1 | 60 | 2.85 | 3.05 | 2500 | 2300 |
| Neutralization | 58.0 | 55 | 2.56 | 3.05 | 1890 | 1550 |
| Total Active Matter after neutralization (%) | | | | | 69.4 | 71.0 |
| Post-ageing | 58.0 | 55 | 2.56 | 3.05 | 1500 | 1350 |
| Batch bleaching | --- | 80 | 2.34 | 2.92 | 65 | 45 |

From Table 5 it is clear that both sulfates are equally effective in improving the conversion of FAME to MES and like sodium sulfate, they also induce a reduction in color during the post ageing step.

## Claims

1. A process for the production of salts of fatty acid alkyl ester sulfonates comprising the steps of:
(a) sulfonating a fatty acid methyl ester with gaseous sulfur trioxide in a continuous process thereby generating a crude sulfonation product comprising more than 15 % by weight of residual fatty acid methyl ester;
(b) dissolving an amount of less than 10% by weight of inorganic sulfates, calculated on the weight of the crude sulfonation product, into the crude sulfonation product resulting from step a);
(c) ageing the solution resulting from step b);
(d) re-esterifying the aged product resulting from step c) by the addition of methanol;
(e) neutralising the re-esterified product resulting from step d); and
(f) bleaching the neutralised product resulting from step e);
wherein up to 25% by weight of the solution resulting from step a) and/or step b) is recycled to and mixed into the fatty acid methyl ester stream being sulfonated in step a).

2. The process according to claim 1 in which the inorganic sulfates dissolved in step b) comprise acid sulfates.

3. The process according to claim 1 or claim 2 in which the inorganic sulfates dissolved in step b) comprise magnesium sulfate and/or sodium sulphate.

4. The process according to any one of claims 1 - 3, wherein the inorganic sulfates are acidic sulfates.

5. The process according claim 4, wherein the acidic sulphate is sodium hydrogen sulphate or acidic magnesium sulphate.

6. The process according to any one of claims 1 - 5 in which the neutralised product resulting from step e) is subjected to a further ageing treatment.

7. The process according to claim 6 in which the aged product resulting from said further ageing treatment is bleached with a bleaching agent.

8. The process according to claim 7, wherein the bleaching agent is hydrogen peroxide or sodium hypochlorite.

9. The process according to claim 6 in which the duration of said further ageing treatment is at least 1 hour.

10. The process according to any one of claims 1 - 9, said process comprising a step b1), wherein at least a part of the inorganic sulfates are mixed with a side stream formed from 1 to 30 wt.% of the total aged product resulting from step c), wherein the formed mixture is recycled to ageing step c).

11. The process according to any one of claims 1 - 10, wherein the process is a continuous process.

12. The process according to any one of claims 1 - 11, wherein the temperature of the reaction mixture of step a) is within the range of 50°C to 80°C.

13. The process according to any one of claims 1 - 12, wherein the molar excess of SO₃ with respect to the fatty acid methyl ester is 10 to 25%.

14. The process according to any one of claims 1 - 13, wherein in step c) the temperature of said solution is raised to 80°C and said solution is aged for 2 hours.

15. The process according to any one of claims 1 - 14, wherein in step d) the temperature is lowered to 60°C.

16. The process according to any one of claims 1 - 15, wherein in step f) the temperature is 60° to 80°C.

17. The process according to anyone of claims 1 - 16, wherein in step f) the pH is 6.5 to 7.0.

## Patentansprüche

1. Verfahren zur Herstellung von Salzen von Fettsäurealkylester-sulfonaten, dass die Schritte:
(a) Sulfonieren eines Fettsäuremethylesters mit gasförmigem Schwefeltrioxid in einem kontinuierlichen Verfahren, wodurch ein rohes Sulfonierungsprodukt erzeugt wird, das mehr als 15 Gew.-% restlichen Fettsäuremethylester umfasst,
(b) Lösen einer Menge von weniger als 10 Gew.-% anorganischer Sulfate, berechnet auf das Gewicht des rohen Sulfonierungsprodukts, in dem aus Schritt a) resultierenden rohen Sulfonierungsprodukt;
(c) Altern der aus Schritt b) resultierenden Lösung;
(d) Erneutes Verestern des aus Schritt c) resultierenden gealterten Produkts durch Zugabe von Methanol;
(e) Neutralisieren des aus Schritt d) resultierenden erneut veresterten Produkts; und
(f) Bleichen des aus Schritt e) resultierenden neutralisierten Produkts;
bei dem bis zu 25 Gew.% von der Lösung aus Schritt a) und/oder Schritt b) zurückgeführt zu und gemischt wird mit dem in Schritt a) sulfonierten Fettsäuremethylesterstrom.

2. Verfahren nach Anspruch 1, bei dem die in Schritt b) aufgelösten anorganischen Sulfate saure Sulfate umfassen.

3. Verfahren nach Anspruch 1 oder 2, bei dem die in Schritt b) aufgelösten anorganischen Sulfate Magnesiumsulfat und/oder Natriumsulfat umfassen.

4. Verfahren nach einem der Ansprüche 1 bis 3, bei dem die anorganischen Sulfate saure Sulfate sind.

5. Verfahren nach Anspruch 4, bei dem das saure Sulfat Natriumhydrogensulfat oder saures Magnesiumsulfat ist.

6. Verfahren nach einem der Ansprüche 1 bis 5, bei dem das aus Schritt e) resultierende neutralisierte Produkt einer weiteren Alterungsbehandlung unterzogen wird.

7. Verfahren nach Anspruch 6, bei dem das aus der weiteren Alterungsbehandlung resultierende gealterte Produkt mit einem Bleichmittel gebleicht wird.

8. Verfahren nach Anspruch 7, bei dem das Bleichmittel Wasserstoffperoxid oder Natriumhypochlorit ist.

9. Verfahren nach Anspruch 6, bei dem die Dauer der weiteren Alterungsbehandlung mindestens 1 Stunde beträgt.

10. Verfahren nach einem der Ansprüche 1 bis 9, bei dem das Verfahren einen Schritt b1) umfasst, bei dem mindestens ein Teil der anorganischen Sulfate mit einem Seitenstrom gemischt werden, der aus 1 bis 30 Gew.% des gesamten aus Schritt c) resultieren gealterten Produkts gebildet wird, wobei die gebildete Mischung zum Alterungsschritt c) zurückgeführt wird.

11. Verfahren nach einem der Ansprüche 1 bis 10, bei dem das Verfahren ein kontinuierliches Verfahren ist.

12. Verfahren nach einem der Ansprüche 1 bis 11, bei dem die Temperatur der Reaktionsmischung von Schritt a) im Bereich von 50°C bis 80°C liegt.

13. Verfahren nach einem der Ansprüche 1 bis 12, bei dem der molare Überschuss an SO₃ in Bezug auf den Fettsäuremethylester 10 bis 25% beträgt.

14. Verfahren nach einem der Ansprüche 1 bis 13, bei dem in Schritt c) die Temperatur der Lösung auf 80°C angehoben wird und die Lösung 2 Stunden lang gealtert wird.

15. Verfahren nach einem der Ansprüche 1 bis 14, bei dem in Schritt d) die Temperatur auf 60°C gesenkt wird.

16. Verfahren nach einem der Ansprüche 1 bis 15, bei dem in Schritt f) die Temperatur 60° bis 80°C beträgt.

17. Verfahren nach einem der Ansprüche 1 bis 16, bei dem in Schritt f) der pH-Wert 6,5 bis 7,0 beträgt.

## Revendications

1. Procédé pour la production de sels de sulfonates d'alkyl-esters d'acides gras comprenant les étapes de :
(a) sulfonation d'un méthylester d'acide gras avec du trioxyde de soufre gazeux dans un procédé continu pour produire un produit de sulfonation brut comprenant plus de 15 % en poids de méthylester d'acide gras résiduel ;
(b) dissolution d'une quantité inférieure à 10 % en poids de sulfates inorganiques, calculée sur le poids du produit de sulfonation brut, dans le produit de sulfonation brut résultant de l'étape a) ;
(c) vieillissement de la solution résultant de l'étape b) ;
(d) ré-estérification du produit vieilli résultant de l'étape c) par l'addition de méthanol ;
(e) neutralisation du produit ré-estérifié résultant de l'étape d) ; et
(f) blanchiment du produit neutralisé résultant de l'étape e) ;
où jusqu'à 25 % en poids de la solution résultant de l'étape a) et/ou de l'étape b) sont recyclés et mélangés dans le courant de méthylester d'acide gras sulfoné dans l'étape a).

2. Procédé selon la revendication 1 où les sulfates inorganiques dissous dans l'étape b) comprennent des sulfates acides.

3. Procédé selon la revendication 1 ou la revendication 2 où les sulfates inorganiques dissous dans l'étape b) comprennent du sulfate de magnésium et/ou du sulfate de sodium.

4. Procédé selon l'une quelconque des revendications 1-3, où les sulfates inorganiques sont des sulfates acides.

5. Procédé selon la revendication 4, où le sulfate acide est l'hydrogénosulfate de sodium ou le sulfate de magnésium acide.

6. Procédé selon l'une quelconque des revendications 1-5 où le produit neutralisé résultant de l'étape e) est soumis à un traitement de vieillissement supplémentaire.

7. Procédé selon la revendication 6 où le produit vieilli résultant dudit traitement de vieillissement supplémentaire est blanchi avec un agent de blanchiment.

8. Procédé selon la revendication 7, où l'agent de blanchiment est le peroxyde d'hydrogène ou l'hypochlorite de sodium.

9. Procédé selon la revendication 6 où la durée dudit traitement de vieillissement supplémentaire est d'au moins 1 heure.

10. Procédé selon l'une quelconque des revendications 1-9, ledit procédé comprenant une étape b1), où au moins une partie des sulfates inorganiques est mélangée avec un courant latéral formé à partir de 1 à 30 % en poids du produit vieilli total résultant de l'étape c), où le mélange formé est recyclé dans l'étape de vieillissement c).

11. Procédé selon l'une quelconque des revendications 1-10, où le procédé est un procédé continu.

12. Procédé selon l'une quelconque des revendications 1-11, où la température du mélange réactionnel de l'étape a) est dans la plage de 50°C à 80°C.

13. Procédé selon l'une quelconque des revendications 1-12, où l'excès molaire de SO₃ par rapport au méthylester d'acide gras est 10 à 25 %.

14. Procédé selon l'une quelconque des revendications 1-13 où, dans l'étape c), la température de ladite solution est augmentée à 80°C et ladite solution est vieillie pendant 2 heures.

15. Procédé selon l'une quelconque des revendications 1-14 où, dans l'étape d), la température est abaissée à 60°C.

16. Procédé selon l'une quelconque des revendications 1-15 où, dans l'étape f), la température est 60° à 80°C.

17. Procédé selon l'une quelconque des revendications 1-16 où, dans l'étape f), le pH est 6,5 à 7,0.
